# EUROPEAN PATENT SPECIFICATION

(11) **EP 2 275 104 B1**
(45) Date of publication and mention of the grant of the patent: **11.05.2016**
(21) Application number: 10006130.8
(22) Date of filing: 14.06.2010
(51) Int. Cl.: A61K 31/4418, A61K 31/663, A61K 38/40, A61K 45/06, A61K 8/06, A61K 8/49, A61K 8/55, A61K 8/98, A61K 8/04, A61Q 5/00, A61P 17/08

(54) **Pharmaceutical and cosmetic composition including lactoferrin, ciclopirox and etidronic acid**
Pharmazeutische und kosmetische Zusammensetzungen mit Lactoferrin, Ciclopirox, und Etidronsäure
Composition pharmaceutique et cosmétique comprenant de la lactoferrine, du ciclopirox et de l'acide étidronique

(30) Priority: 17.06.2009 IT MI20091075
(43) Date of publication of application: 19.01.2011
(73) Proprietor: Valetudo S.r.l., 24030 Presezzo (BG) (IT)
(72) Inventor: Bortolin, Vittorio, 24030 Presezzo ( BG) (IT)
(74) Representative: Lecce, Giovanni

(56) References cited:
- WO-A1-98/02138
- WO-A1-2007/144613
- WO-A1-2008/095796
- WO-A2-2007/054818
- WO-A2-2007/100555
- US-A1- 2008 038 219
- DATABASE WPI Week 200381 Thomson Scientific, London, GB; AN 2003-869502 XP2577924, & JP 2003 277221 A (FUKUDA K) 2 October 2003 (2003-10-02) & FUKUDA T: "the predatory mites is firstly the manual breed of Skin external preparation useful as cosmetics such as skin lotion, contains lactoferrin and beta-1,3 glucan", WPI / THOMSON,, vol. 2003, no. 81, 2 October 2003 (2003-10-02), XP002577924,
- ZAREMBER K A ET AL: "Antifungal activities of natural and synthetic iron chelators alone and in combination with azole and polyene antibiotics against Aspergillus fumigatus", ANTIMICROBIAL AGENTS AND CHEMOTHERAPY, AMERICAN SOCIETY FOR MICROBIOLOGY, WASHINGTON, DC, US, vol. 53, no. 6, 1 June 2009 (2009-06-01), pages 2654-2656, XP002577925, ISSN: 0066-4804, DOI: 10.1128/AAC.01547-08 [retrieved on 2009-03-23]
- DATABASE WPI Week 200353 Thomson Scientific, London, GB; AN 2003-561912 XP2578008, & JP 2003 089629 A (KAKUNAI JUYOTAI KENKYUSHO KK) 28 March 2003 (2003-03-28) & ANDO K ET AL: "Preparation of compositions such as novel cosmetics, external preparation and bathing agent, for treating atopic dermatitis, acne, axillary smell and scalp dandruff, comprises addition of colostrum of mammal as active ingredient", WPI / THOMSON,, vol. 2003, no. 53, 28 March 2003 (2003-03-28), XP002578008,
- SHAPIRO J ET AL: "Medicated shampoos", CLINICS IN DERMATOLOGY, J.B. LIPPINCOTT, PHILADELPHIA, PA, US, vol. 14, no. 1, 1 January 1996 (1996-01-01), pages 123-128, XP002578009, ISSN: 0738-081X
- DE GANNES G C ET AL: "Psoriasis and pustular dermatitis triggered by TNF-[alpha] inhibitors in patients with rheumatologic conditio", ARCHIVES OF DERMATOLOGY, AMERICAN MEDICAL ASSOCIATION, US, vol. 143, no. 2, 1 February 2007 (2007-02-01), pages 223-231, XP002578010, ISSN: 0003-987X
- GURMIN V ET AL: "Psoriasis: Response to high-dose intravenous immunoglobulin in three patients", BRITISH JOURNAL OF DERMATOLOGY, WILEY-BLACKWELL PUBLISHING LTD, GB, vol. 147, no. 3, 1 September 2002 (2002-09-01), pages 554-557, XP002578011, ISSN: 0007-0963, DOI: 10.1046/J.1365-2133.2002.04753.X [retrieved on 2002-09-05]

## Description

The present invention refers to pharmaceutical and cosmetic compositions for topical use comprising Lactoferrin and Ciclopirox, and/or salts thereof, and optionally Etidronic Acid and/or salts thereof.

More in particular, the present invention refers to pharmaceutical and cosmetic compositions comprising at least the first two of the compounds listed above and to the use thereof in the treatment of dermatological disorders or conditions such as seborrheic dermatitis and dandruff. Seborrheic dermatitis is a chronic inflammatory pathology which affects areas of the face, the scalp and the trunk where there is a high presence of sebum glands. The aetiology of seborrheic dermatitis is not clear yet even if some species of lipophilic fungi belonging to the *Malassezia* genus could be the major factors.

Dandruff is instead a pathology that mainly involves the scalp which we assume could have aetiology similar to that of seborrheic dermatitis.

The cosmetic or dermatological treatment options of seborrheic dermatitis and dandruff include the application on the skin or the scalp of formulations containing anti-fungal active ingredients. In fact there are shampoos on the market containing selenium disulfide, zinc pyrithione or ketoconazole and creams or lotions containing terbinafinem, sulfacetamide, ketoconazole or formulations for topical use of said active ingredients with cortisone drugs for the treatment of these conditions/disorders of the skin and scalp.

Ciclopirox or 6-cyclohexyl-1-hydroxy-4-methyl-1.2dihydropyridin-2-one is an antifungal active ingredient of the family of hydroxy pyridones which is finding increasingly greater use in the treatment of seborrheic dermatitis and dandruff, given its particular effectiveness and low toxicity. The antifungal action of ciclopirox is attributed to its chelating capacity of ferric ions, indispensible for the metabolism of fungi of the *Malassezia* genus. **Such a capacity is well known, e.g. from XP 002577925,** Zarember at al., Antimicrobial Agents and Chemiotherapy June 2009, p. 2654-2656 (vol. 53, No. 6**).**

The formulations based on ciclopirox and salts thereof, even though effective in the treatment of seborrheic dermatitis and dandruff, tend to be unstable due to the chemical degradation caused by the ferric ions present in the excipients used for the production of the finished products and in the stainless steel production equipment.

The action of the ciclopirox, In the formulations that contain it, is therefore reduced by the virtually inevitable presence of ferric ions coming from the production systems and the stainless steel production equipment of the pharmaceutical and cosmetic compositions or present in the excipients used in the production of said compositions. The presence of said ions in such pharmaceutical or cosmetic formulations can promote the establishment of mechanisms of resistance of the *Malassezia* to the ciclopirox, with the resulting loss of effectiveness In the treatment of dandruff and seborrheic dermatitis.

Lactoferrin is a glycoprotein, normally present in milk and tear secretions, capable of binding the ferric ions and at the same time of providing a discreet anti-inflammatory action.

From the scientific article XP-002577924, WPI - Thomson Scientific, London GB, from WO2007/100555 A2 (Ventria Bioscience) September 7, 2007 and from WO2007/144613 A1 (Sinclair Pharmaceuticals Limited) December 21, 2007 it is well known that Lactoferrin acts as an antimicrobial viz antifungal agent.

Moreover, from the scientific article XP-002577925 Zaramber et al., Antimicrobial Agents and Chemiotherapy June 2009, p. 2654-2656 it is well known that such action accurs by iron deprivation and from WO98/02138 A1 (Cosmeticos Natural Ind. Com.) January 22, 1998 Lactoferrin is Commonly known as chelating agent for treating seborrheic dermatitis.

Etidronic acid, compound known by the chemical name (1-hydroxy-1-phosphono-ethyl) phosphonic acid is a chelating agent of the ferric ions currently used in the technical-industrial sector.

Etidronic acid is also known to be useful in treatments of psorlasis, e.g. from De Gannes G. C. et al "Psorlasis and pustular dermatitis triggered by TNF-α inhibitors in patients with rheumatologic conditions" Archives of Dermatology/vol. 143, n. 2, Feb. 2007, p. 223-231 and from Gurmin at al "Psoriasis: Response to high-dose intravenous immunoglobulin in three patients" British Journal of Dermatology 2002, vol. 147, no. 3, 2002, p. 554-557.

It was surprisingly found that compositions containing at least ciclopirox and/or salts thereof and lactoferrin, and optionally etidronic acid and/or salts thereof, overcome the problems reported above and provide an unexpected or in any case a very enhanced antifungal action.

The object of the present invention is therefore to provide a composition for topical use which includes at least ciclopirox and/or salts thereof and lactoferrin, and optionally etidronic acid and/or salts thereof, and a physiologically acceptable carrier.

The term "for topical use" as used herein means applied to the skin or the scalp,

By "Ciclopirox and/or salts thereof" we mean Ciclopirox optionally as salt of alkali metals for example of sodium or potassium; as ammonium salt; as primary amine salt as for example C₁-C₈ alkyl amine; secondary as for example C₁-C₈ dialkyl amine; or tertiary as for example C₁-C₈ trialkyl amine; diamine; alkanolamine salts, as for example C₁-C₈ alkanolamine or C₁-C₈ dialkanolamine or C₁-C₈ for example C₁-C₈ trialkyl amine; diamine; alkanolamine salts, as for example C₁-C₈ alkanolamine or C₁-C₈ dialkanolamine or C₁-C₈ trialkanolamine; C₁-C₈ dialkyl C₁-C₈ alkanolamine; C₁-C₈ alkyl C₁-C₈ dialkanolamine. Preferably, a Ciclopirox salt is Ciclopirox Olamine. By "Etidronic Acid and/or salts thereof" we mean Etidronic Acid optionally as alkali metal salts for example of sodium or potassium; as ammonium salt; as primary, secondary or tertiary amine salt.

The compositions of the present invention comprise Ciclopirox or one of salts thereof, preferably Ciclopirox Olamine, and Lactoferrin; or Lactoferrin, Ciclopirox or one of salts thereof, and Etidronic Acid or one of salts thereof and a physiologically acceptable carrier.

The compositions of the present invention preferably comprise Ciclopirox Olamine and Lactoferrin in a ratio from 8:1 to 1:20. Even more preferably, the compositions of the present invention comprise Lactoferrin, Ciclopirox Olamine, and Etidronic Acid and a physiologically acceptable carrier. The compositions of the present invention may be in the form of oil emulsion in water or water in oil including an oily phase and an aqueous phase, or in the form of gel, spray, shampoo, solution for example for tissues or water or hydroalcoholic-based lotion or pressurized foam and are object of the present invention.

In the oil emulsions or in water or water in oil, the oily phase can contain branched or non-branched hydrocarbons as for example oil of vaseline, paraffin, squalene, polyisobutylene, optionally hydrogenated polydecene, preferably oil of Vaseline and hydrogenated polydecene; silicon oils, for example dimethicone, phenyl trimethicone, cyclomethicone and dimethiconol; fatty acids selected from saturated fatty acids and partially unsaturated which have between 8 and 20 atoms of carbon, for example lauric, myristic, palmitic, stearic, isostearic, oleic, linoleic, eicosanoic, docosanoic, erucic; C₁-C₂₀ alkyl and alkenyl esters of fatty acids saturated and partially unsaturated which have between 10 and 30 atoms of carbon for example decyl oleat, isodecyl oleate, dioctyl maleate, isopropyl palmitate, isoesyl palmitate, ethylesyl palmitate, lauryl lactate, myristyl lactate, cetyl lactate, isostearyl neopentanoate, ethylesyl isostearate, myristyl myristate, esyl laurate, cetyl palmitate, isopropyl palmitate, hexadecyl stearate, decyl stearate, isopropyl isostearate, diisopropyl adipate, diisoesyl adipate, diesyldecyl adipate, isocetyl stearoyl stearate, C₁₂-C₁₅ alkyl lactate, cetearyl isononanoate and mixtures of these, preferably ethylesyl palmitate, decyl oleate, isopropyl myristate, isopropyl palmitate, myristyl myristate, isostearyl neopentanoate, cetearyl isononanoate.

The oily phase can furthermore contain cetylic alcohol, stearylic alcohol, glyceryl stearate, polysorbates, esters or ethers of fatty acids from C₁₂ to C₁₈ with polyethylene glycols.

The oily phase can also contain esters of fatty acids between C₁₂ and C₂₀ glycerol and polyglycerol, vitamins such as vitamin E and ureides such as allantoin.

The components of the aqueous phase can include water, alcohols such as denatured ethyl alcohol or isopropyl alcohol, organic salts, for example sodium citrate, potassium sodium tartrate, potassium sorbate, sodium benzoate and ethylenediaminetetraacetic acid (EDTA); inorganic salts such as sodium chloride, sodium metabisulfite, magnesium sulphate; buffering agents, for example sodium hydroxide, sodium bicarbonate, sodium hydrogen phosphate, and mixtures of these; organic acids such as for example salicylic or citric acid; preservatives like parabens, as for example methylparaben, phenoxyethanol, chlorhexidine, chlorphenesin, imidazolidinyl urea, derivatives of glycine; essential oils as for example eucalyptus, thyme, cinnamon, geranium; amino acids, as for example arginine, betaine and lysine, and their derivatives; jellying agents, for example derivatives of cellulose, alginates, carrageenin, derivatives of guar gum, polymers of acrylic acids as for example polyacrylates, methacrylates, carbomer; vitamins such as for example niacinamide.

The aqueous phase can further comprise glycerol, propylene glycol, butylenes glycol, ethyl alcohol, isopropyl alcohol, polyalcohols, amino alcohols as for example methanolamine, saccharic components derivatives of amide and cellulose.

Preferably, the water used in the present invention is deionized water. The water content in the preparation of the present invention is relative to the total content of the other components used so that the total weight of the preparation is equal to 100% by weight of the topical preparation.

The compounds with sequestering property of the ferric ions, that is, Ciclopirox or salts thereof, Etidronic Acid or salts thereof, Lactoferrin are in an amount of between 0.5 and 12% by weight, preferably between 1 and 8% by weight of the preparation.

Etidronic acid is allowed in products to be rinsed such as for example shampoos and hair cream to be rinsed, at a maximum concentration of 1.5%.

The emulsions of the present invention can include, if desired, a vast range of optional components, as for example fragrances, plant extracts, solar filters, colouring agents. These optional components can be added to the preparation after the oily and aqueous phases have been combined or mixed together.

The method of preparation of the compositions for topical application, on the surface of the skin of mammals in particular human skin, for cosmetic and/or pharmaceutical use of the present invention include mixing the agents with sequestering property of the ferric ions with a physiologically acceptable carrier.

By the term "physiologically acceptable" as used here, we mean compatible with the skin or the scalp, non toxic, non reactive.

The emulsions of the present invention can be prepared by any method known in the art. In general, such methods of preparation include the passage of the oily phase and aqueous phase preparation in separate containers and then the combination of the two phases.

The oily phase is obtained by mixing the components of the oily phase through constant shaking in a mixing container separate from the mixing container which contains the components of the aqueous phase, for a period of time that depends on the size of the mixing container used, and thus the appropriate mixing time can vary from a few minutes to hours. If required, in order to allow the solid components to dissolve, the oily phase can be heated to a temperature of about 40° to 90 °C, preferably between 60° and about 75 °C.

The aqueous phase is obtained by dissolving solid and liquid components in water and shaking gently, for a period of time that depends on the size of the mixing container used and so the adequate mixing time can vary from a few minutes to hours.

If necessary, to obtain the emulsion, the aqueous phase can be heated to the same dissolving temperature of the oily phase, before mixing the two phases.

The oil phase is then mixed together with the aqueous one with constant shaking until it becomes homogeneous. The mixing of the two phases can occur in the same container or tank in which the aqueous or oily phase was originally mixed.

The resulting mixture is cooled, for example by means of circulation of cold water in the hollow space of the container wherein the preparation is being made until the mixture reaches the desired temperature. If required, a component as defined above can be added to the mixture during or after the cooling phase under constant slow mixing. At the end of the process, the mixture can be stored in storage tank and then packaged in the final containers.

The gels of the present invention may consist of various components, part of which are common to the emulsions and previously mentioned or listed in the examples.

The gels of the present invention can be prepared by any method known in the art. In general, these methods provide for the preparation of an aqueous phase which is then gelatinized. The aqueous phase is obtained by dissolving solid and liquid components in water and shaking, for a period of time that depends on the intensity of the shaking and on the size of the mixing container used; the adequate mixing time can therefore vary from a few minutes to hours. The aqueous phase is gelatinized with a jellying agent which can be added to the aqueous phase while being shaken or at the end of agitation. The preparation also provides for the control and adjustment of the final pH of the product.

The lotions, sprays, shampoos and solutions of the present invention may consist of various components, part of which are common to the emulsions and previously mentioned or listed in the examples.

Said formulations can be prepared by any method known in the art. These methods provide for the preparation of an aqueous phase which, depending on the cases, is appropriately perfumed, coloured and thickened. The aqueous phase is obtained by dissolving solid and liquid components in water and shaking, for a period of time that depends on the intensity of the shaking and on the size of the mixing container used, and thus the adequate mixing time can vary from a few minutes to hours. The aqueous phase can be perfumed, coloured and/or thickened with perfumes, colouring or thickeners which can be added to the aqueous phase during the shaking process or at the end of this process. The preparation also provides for the control and adjustment of the final pH of the product.

A further object of the present invention is the use of the compositions mentioned above for the preparation of a medicament for the treatment of seborrheic dermatitis.

A further object of the present invention is the use of the compositions listed above in the cosmetic treatment of dandruff.

In the range of formulations and compositions of the present invention, different ingredients for cosmetic and/or pharmaceutical use can be employed.

Both the pharmaceutical type preparations and the cosmetic ones can be in the form of cream, shampoo, solution, lotion or sprays for the skin. In these cases, all the ingredients acknowledged as suitable for pharmaceutical and/or cosmetic products can be introduced into the physiologically acceptable carrier and be used to prepare the creams, shampoos, the solution, lotions, gels or sprays for skin, which fall within the scope of application of the present invention.

In order to better describe the present invention, we provide some examples.

The formulation examples listed below describe only some of the possible compositions of the present invention. While these examples use formulations selected, according to the present invention, it is clear that these examples are illustrative and not limiting. It is also noted that all the Ciclopirox salts, detailed above, can be used in accordance with the instructions of the present invention, in formulations similar to those provided below.

All the components of the compositions of the present invention are commercially available and can be easily prepared by following procedures know in the art.

The ingredient "Aqua" cited in the examples provided below is always deionized water.

All the parts, percentages and proportions referred to and provided in the claims are understood to be by total weight of the composition, unless otherwise indicated.

The names of the ingredients used and listed in the examples follow the International Nomenclature of Cosmetic ingredients (INCI) and are those officially recognized in the cosmetic sector.

### Example 1

### Cream for skin (emulsion without emulsifier) - % by weight

Part A: Hydrogenated Polydecene 6.00 Isopropyl Palmitate 3.00; Part B Ammonium Acryloyldimethyltaurate/VP Copolymer 1.00; Part C Aqua 74.20 Glycerin 4.00 denat. alcohol 10.00 Lactoferrin 1.00 Ciclopirox Olamine 0.50; Part D Parfum 0.30.

Preparation:
I) Mix Part A and Part B at room temperature;
II) Add while shaking Part C to the mixture as listed in Step I) and then add Part D;
III) Homogenize the emulsion thus obtained.

### Example 2

### Face cream - % by weight

Part A Ceteareth-25 2.00 Ceteareth-5 3.00 Glyceryl Stearate 2.00 Cetyl Alcohol 2.00 Caprylic/Capric Triglyceride 7.00 Dimethicone 0.50 Isopropyl Palmitate 5.00 Tocopheryl Acetate 0.50; Part B Aqua 69.00 Carbomer 0.20; Part C Sodium Hydroxide (10% in water) 0.80 Phenoxyethanol 0.50 Methylparaben 0.20; Part D Glycerin 5.00 Lactoferrin 1.50 Ciclopirox Olamine 0.50 Parfum 0.30

Preparation:
I) Melt Part A at around 70° C, then add Part B;
II) Heat Part C at around 70°C;
III) Add what obtained in Step II) to what obtained in Step I) and cool to room temperature while shaking;
IV) Add Part D to what obtained in Step III) at about 35°C;
IV) Homogenize the emulsion.

### Example 3

### Solution for imbued tissues - % by weight

Part A PEG-7 Glyceryl Cocoate 2.50 PEG-40 Hydrogenated Castor Oil 1.00 Polysorbate-20 1.00 Glycerin 7.50 Denat. alcohol 16.00 Ciclopirox Olamine 1.50 Perfume 0.50; Part B Aqua 68.00 Lactoferrin 1.00 Citric Acid (10% in water) 0.90 Disodium EDTA 0.10

Preparation: Add Part B and Part A at room temperature and mix until a clear solution is obtained.

### Example 4

### Rinsing hair cream - % by weight

Part A Behentrimonium Chloride 2.00 Ceteareth-25 1.50 Cetearyl alcohol 2.00 Paraffinum liquidum 2.00; Part B Aqua 85.2; Part C Glycerin 5.00 Lactoferrin 0.50 Ciclopirox Olamine 1.50 Parfum 0.30; Part D Citric Acid as necessary to obtain a pH between 6.00 and 6.50.

Preparation:
I) Melt Part A at 75°C;
II) Heat Part B at 75°C;
III) Add what was obtained in II) to that obtained in I) while shaking;
IV) Cool while shaking the mixture as per Step III);
V) At 35°C add the components of Part C to that obtained in IV);
VI) Adjust the pH with Part D taking it to values between 6.00 and 6.50.

### Example 5

### Shampoo - % by weight

Part A Guar Hydroxypropyltrimonium Chloride 0.30; Part B Aqua 40.30; Part C Sodium Laureth Sulfate (solution at 28%) 42.90 Sodium Lauroamphoacetate 6.70 Parfum 0.40 Ciclopirox Olamine 1.50 Etidronic Acid 0.20 Cocamidopropyl Betaine 6.70; Part D Citric Acid (25% in water) as needed to bring the pH to 5.5; Part E Sodium Chloride 1.00

Preparation:
I) Dissolve Part A in Part B at room temperature;
II) Add one after the other the components of Part C to that which was obtain in Step I);
III) Adjust the pH of the mixture thus obtained to 5.5 by adding Part D;
IV) Adjust the viscosity of the mixture by adding Part E.

### Example 6

### Conditioning Shampoo - % by weight

Part A Polyquaternium-7 1.50; Part B Aqua 37.00; Part C Sodium Laureth Sulfate (solution 28%) 42.90 Sodium Lauroamphoacetate 6.70 Parfum 0.40 Lactoferrin 1.50 Ciclopirox Olamine 2.00 Etidronic Acid 0.30 Cocamidopropyl Betaine 6.70; Part D Citric Acid (25% in water) as needed to reach a pH of 5.5; Part E Sodium Chloride 1.00

Preparation:
I) Dissolve Part A in Part B at room temperature;
II) Add to the mixture obtained in Step I) the components of Part C;
III) Adjust the pH of the mixture covered in Step II) to 5.5 with Part D;
IV) Adjust the viscosity of the mixture thus obtained by adding Part E.

### Example 7

### Face cleansing gel - % by weight

Part A Glycerin 10.00 PEG-8 5.00 Panthenol 0.50 Phenoxyedianol 0.50 Methylparaben 0.25 denat. alcohol 7.50 Parfum 0.20 Etidronic Acid 0.20 Lactoferrin 0.80 Allantoin 0.10 Niacinamide 0.10; Part B Aqua 74.15; Part C Ammonium Acryloyldimethyltaurate/VP Copolymer 0.70.

Preparation:
I) Dissolve Part A in Part B while shaking;
II) Add Part C to the mixture obtained in Step I) and continue shaking until homogenized.

### Example 8

### Hair lotion - % by weight

Part A Aqua 51.30 PEG/PPG-18/18 Dimethicone 0.30; Part B Lactoferrin 1.50 Benzophenone-4 0.20; Part C Isopropyl Alcohol 45.00 Parfum 0.30; Part D Niacinamide 0.30 Cetrimonium Chloride 0.50 Panthenol 0.20 Polyquaternium-7 0.10 Ciclopirox Olamine 0.30; Part E Citric Acid as needed to reach a pH between 6.0 and 6.5.

Preparation:
I) Mix the components of Part A with intense shaking;
II) Mix while shaking intensely the components of Part B and add the mixture to that obtained in Step I;
III) Mix the components of Part C together and add the mixture obtained in Step III) to that obtained in Step II);
IV) Add one after the other the components of Part D to that which was obtain in Step III);
V) Adjust the pH of the mixture in Step IV) to 6.0 - 6.50 by adding Part E.

### Example 9

### Face cream - % by weight

Part A: Polysorbate 80 4.50 Cetearyl Alcohol 18,00 Cholesterol 0.45 Tocopherol 0.02 Ascorbyl Palmitate 0.01 Lecithin 0.01 Citric Acid 0.01; Part B: Aqua 58.40 Propylene Glycol 9.00 Salicylic Acid 0.50 Methylparaben 0.10; Part C Aqua 7.00 Lactoferrin 1.50 Ciclopirox Olamine 0.50. Preparation:
I) Heat Part A at 74°C and Part B at 75°C;
II) Slowly add Part B to Part A while shaking;
III) Continue shaking the mixture and cool it to 50°C;
IV) At 50°C add Part C;
V) Continue to cool the mixture being shaken until reaching room temperature. The cream thus obtained that is washable with water has a final pH of between 6.0-7.0.

### Example 10

### Lotion for face and body - % by weight

Part A: Lactoferrin 2.00 Aqua 49.40; Part B: Denat. alcohol, 46.00 Ciclopirox Olamine 0.60 Salicylic Acid 1.80 Triethanolamine 0.20.

Prepare separately, through simple mixing of the ingredients Part A and Part B. Pour Part A in Part B and mix. Final pH: 4.0 - 5.0.

### Example 11

It has been verified that the formulations provided in the present invention are capable of inhibiting the microbial growth by studying in particular the antimycotic action of CICLOPIROXOLAMINE alone and in association with LACTOFERRIN.

To assess the capacity of the LACTOFERRIN to enhance the antimycotic action of the CIPROPIROXOLAMIN, a suspension of a MALASSEZIA GLOBOSA STRAIN is inoculated on a specific culture medium and the capacity of the compounds to inhibit the mycotic growth is observed, by counting of the UFC/ml.

As first approach, the two compounds are tested at the concentration of 1:500 and, subsequently, diluted in culture medium at 2 decreasing concentrations (10x and 100x) in order to visualize the individual activities. Then in order to evaluate the capacity of the LACTOFERRIN to increase the antimycotic effect of the CICLOPIROXOLAMIN, the same test was carried out on the mix of the two active ingredients at different ratios (1:2 and 1:5).

Each test was performed three times and the incubation times were 1 and 4 hours respectively.

Based on the results obtained, the best dilution for the execution of the tests was the 10X dilution and both the incubation times (1 hour and 4 hours) were taken into consideration since the experimental conditions permitted an accurate count to be carried out of the colonies on the Petri dishes.

Based on the results obtained from the evaluation of the mycotic growth after treatment, it can be noted how the CICLOPIROXOLAMINE is decidedly more effective in reducing the growth compared to the LACTOFERRIN.

The results obtained from the comparison of the inhibition of the mycotic growth just through CICLOPIROXOLAMINE and its combination with LACTOFERRIN in the 2 different ratios (1:2 and 1:5), demonstrate an effective increase in the effectiveness of the CICLOPIROXOLAMINE by the LACTOFERRIN.

This increase is particularly marked if the strain of Malassezia Globosa is inoculated with the mixture in the ratio 1:5 (concentration of the LACTOFERRIN 5 times greater compared to the CICLOPIROXOLAMINE). The effectiveness of the LACTOFERRIN in enhancing the action of the CICLOPIROXOLAMINE is > 60% (compared to the action of the CICLOPIROXOLAMINE alone) already after the first hour of incubation and this increase is confirmed also by a longer incubation time (4 hours).

### Execution of the Test:

The microorganism used is MALASSEZIA GLOBOSA.

The microorganisms were cultured in specific culture medium (Dixon's Agar) until reaching a concentration of approximately 105 vital cells per ml. Aliquots of 1 ml of this inoculum were then taken to which were added respectively:
- Ciclopiroxolamine 1:500
- Lactoferrin 1:500
- Mixture 1:2 Ciclopiroxolamine:lactoferrin
- Mixture 1:5 Ciclopiroxolamine:lactoferrin

As positive control, the untreated inoculum was used (MALASSEZIA and diluting liquid), as negative controls the two active ingredients in diluting liquid.

After incubations of 1 hour and 4 hours respectively, aliquots of 200 µl of each suspension were spread on Petri dishes.

For each treatment, the test was carried out three times and for 3 different dilutions (non diluted suspension (TQ), dilution 10x and dilution 100X).

The 54 dishes obtained were then examined, after incubation of 5-6 days and the microbial load of each specimen was calculated (UFC/ml).

### Criteria of evaluation:

The reduction in the time of the number of microbial colonies that developed (UFC/ml) indicates that the product tested has antimycotic property.

### The % increase of the antimycotic property corresponds to the % reduction of the growth of Malassezia Globosa

### Results of the Test:

**DIAGRAM 1**

| | **Period of incubation (1 hour)** | | | | | |
|---|---|---|---|---|---|---|
| | **REDUCTION OF MALASSEZIA GLOBOSA CELLS GROWTH** | | | | | |
| | **UFC/ml** | | | | | **%** |
| | TQ | dil 10X | X50* | AVERAGE | Log (AVERAGE) | |
| A - CICLOPIROXOLAMINE 1:500 | CC | 208 | 10400 | 9666.667 | 3.985276743 | 0 |
| | CC | 170 | 8500 | | | |
| | CC | 202 | 10100 | | | |
| B- LACTOFERRIN 1:500 | CC | 250 | 12500 | 13483.33 | 4.129797271 | -39.4 |
| | CC | 303 | 15150 | | | |
| | CC | 256 | 12800 | | | |
| A:B(1:2) | CC | 320 | 16000 | 11833.33 | 4.073107098 | -22.4 |
| | CC | 242 | 12100 | | | |
| | CC | 148 | 7400 | | | |
| A:B(1:5) | CC | 67 | 3350 | 3266.667 | 3.514104821 | 66.2 |
| | CC | 63 | 3150 | | | |
| | CC | 66 | 3300 | | | |

**DIAGRAM 2**

| **Period of incubation (4 hour)** | | | | | | |
|---|---|---|---|---|---|---|
| | **REDUCTION OF MALASSEZIA GLOBOSA CELLS GROWTH/** | | | | | |
| | **UFC/ml** | | | | | **%** |
| | TQ | dil 10X | X50* | AVERAGE | Log (AVERAGE) | |
| A-CICLOPIROXOLAMINE 1:500 | CC | 38 | 1900 | 1616.667 | 3.208620573 | 0 |
| | CC | 34 | 1700 | | | |
| | CC | 25 | 1250 | | | |
| B- LACTOFERRIN 1:500 | CC | 200 | 10000 | 12866.67 | 4.109466162 | -695.8 |
| | CC | 340 | 17000 | | | |
| | CC | 232 | 11600 | | | |
| A:B(1:2) | CC | 51 | 2550 | 2633.333 | 3.420505782 | -62.8 |
| | CC | 55 | 2750 | | | |
| | CC | 52 | 2600 | | | |
| A:B(1:5) | CC | 11 | 550 | 633.3333 | 2.801632118 | 60.82 |
| | CC | 12 | 600 | | | |
| | CC | 15 | 750 | | | |

| | | | | | | |
|---|---|---|---|---|---|---|
| *calculation made based on the dilution (200 µl of suspension diluted 10X on Petri dish) | | | | | | |

The % of reduction of the growth of Malassezia Globosa corresponds to the % increase of the antimycotic property of the substances tested.

As can be inferred from Diagram 1, shown above, after one hour of incubation a marked reduction in the growth of Malassezia Globosa by using CICLOPIROXOLAMINE (1:500) is noted. This reduction is greatly enhanced by the LACTOFERRIN (1:500) used in the ratio of 1:5.

The increase in the antimycotic activity (corresponding to the % of inhibition of the growth of Malassezia Globosa) is equal to 66.2% compared to the CICLOPIROXOLAMINE as such.

As can be inferred from Diagram 2, shown above, after four hours of incubation a marked reduction in the growth of Malassezia Globosa by using CICLOPIROXOLAMINE (1:500) is noted. This reduction is greatly enhanced by the LACTOFERRIN (1:500) used in the ratio of 1:5.

The increase in the antimycotic activity (corresponding to the % of inhibition of the growth of Malassezia Globosa) is equal to 60.82 % compared to the CICLOPIROXOLAMINE as such. From the results of the test shown above, we can affirm that CICLOPIROXOLAMINE has a marked antimycotic activity towards Malassezia Globosa compared to LACTOFERRIN. Surprisingly, the use of LACTOFERRIN in combination with CICLOPIROXOLAMINE, in the ratio of 1:5, demonstrated an increase in the antimycotic action of the latter (increase % of the antimycotic activity, corresponding to the reduction % of the mycotic growth equal to 66.2% after 1 hour of incubation).

A similar effect was also observed in case of compositions including LACTOFERRIN and ETIDRONIC ACID or salts thereof; CICLOPIROXOLAMINE and ETIDRONIC ACID or salts thereof; CICLOPIROXOLAMINE, LACTOFERRIN and ETIDRONIC ACID or salts thereof.

For this reason, the compositions given in the present invention are particularly suitable for use in the treatment of dandruff and seborrheic dermatitis.

## Claims

1. A composition for topical use comprising two compounds selected in the group comprising Ciclopirox and Lactoferrin in a ratio from 8:1 to 1:20, and a physiologically acceptable carrier.

2. A composition as claimed in claim 1, **characterized by** further comprising Etidronic Acid and/or salts thereof.

3. A composition as claimed in claims 1 or 2 as oil in water or water in oil emulsion, gel, spray, shampoo, solution, lotion.

4. A composition as claimed in claim 1 or 2, **characterized by** the fact that said compounds are in amount comprised between 0.5 and 12%, preferably between 1 and 8% by weight of the composition.

5. Use of a composition according to claims 1 to 4 for the preparation of a medicament for the treatment of Seborrheic dermatitis.

6. Use of a composition according to claims 1 to 4 for the cosmetic treatment of dandruff.

## Patentansprüche

1. Zusammensetzung zur topischen Anwendung, umfassend zwei Verbindungen, die aus der Gruppe ausgewählt sind, die Ciclopirox und Lactoferrin in einem Verhältnis von 8:1 bis 1:20 umfasst und einen physiologisch akzeptablen Träger.

2. Zusammensetzung nach Anspruch 1, **dadurch gekennzeichnet, dass** sie ferner Etidronsäure und/oder Salze davon umfasst.

3. Zusammensetzung nach Anspruch 1 oder 2 als Öl-in-Wasser- oder Wasser-in-Öl-Emulsion, Gel, Spray, Shampoo, Lösung, Lotion.

4. Zusammensetzung nach Anspruch 1 oder 2, **gekennzeichnet durch** die Tatsache, dass die Verbindungen in einer Menge vorliegen, die zwischen 0,5 und 12%, vorzugsweise zwischen 1 und 8% des Gewichts der Zusammensetzung liegt.

5. Verwendung einer Zusammensetzung nach den Ansprüchen 1 bis 4 für die Zubereitung eines Medikaments für die Behandlung von seborrhoischer Dermatitis.

6. Verwendung einer Zusammensetzung nach den Ansprüchen 1 bis 4 für die kosmetische Behandlung von Schuppen.

## Revendications

1. Une composition à usage topique comprenant deux composés sélectionnés dans le groupe comprenant le Ciclopirox et la Lactoferrine dans un rapport allant de 8/1 à 1/20, et un véhicule physiologiquement acceptable.

2. Une composition telle que revendiquée dans la revendication 1, **caractérisée en ce qu'**elle comprend en outre de l'acide étidronique et/ou des sels de celui-ci.

3. Une composition telle que revendiquée dans les revendications 1 ou 2 comme émulsion huile dans eau ou eau dans huile, gel, spray, shampooing, solution, lotion.

4. Une composition telle que revendiquée dans la revendication 1 ou la revendication 2, **caractérisée par le fait que** lesdits composés sont en quantité comprise entre 0,5 et 12 %, préférablement entre 1 et 8 % en poids de la composition.

5. Utilisation d'une composition selon les revendications 1 à 4 pour la préparation d'un médicament pour le traitement de la dermatite séborrhéique.

6. Utilisation d'une composition selon les revendications 1 à 4 pour le traitement cosmétique des pellicules.
